(19) Europäisches Patentamt
European Patent Office
Office européen des brevets

(11) Numéro de publication: **0 021 923**
**B1**

(12) **FASCICULE DE BREVET EUROPEEN**

(45) Date de publication du fascicule du brevet: **07.09.83**

(51) Int. Cl.³: **A 61 K 31/395**, C 07 D 461/00, C 07 D 471/14 // (C07D471/14, 221/00, 221/00, 209/00)

(21) Numéro de dépôt: **80400807.6**

(22) Date de dépôt: **06.06.80**

(54) **Procédé de préparation de dérivés vincaminiques et dérivés vincaminiques.**

(30) Priorité: **22.06.79 FR 7916030**

(43) Date de publication de la demande:
**07.01.81 Bulletin 81/1**

(45) Mention de la délivrance du brevet:
**07.09.83 Bulletin 83/36**

(84) Etats contractants désignés:
**AT BE CH DE FR GB IT LI LU NL SE**

(56) Documents cités:
**FR-A-2 190 113**

(73) Titulaire: **SYNTHELABO, 58 rue de la Glacière, F-75013 Paris (FR)**

(72) Inventeur: **Rossey, Guy, 15, rue des Amandiers, F-94230 Cachan (FR)**

(74) Mandataire: **Thouret-Lemaitre, Elisabeth, Service Brevets - SYNTHELABO 58, rue de la Glacière, F-75621 Paris Cedex 13 (FR)**

**EP 0 021 923 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Procédé de préparation de dérivés vincaminiques

La présente invention concerne une nouvelle synthèse de dérivés vincaminiques en particulier de la vincamine, de l'apovincamine et de la désoxyvincamine et des esters éthyliques vincaminique, apovincaminique et désoxyvincaminique, sous la forme de racémates et d'énantiomères.

Plusieurs synthèses de la vincamine ont déjà été décrites dans la littérature; il s'agit soit d'hémisynthèses à partir d'alcaloïdes naturels (p. e. à partir de la tabersonine, brevet français 2 184 119) soit de synthèses totales (brevet français 2 081 593; brevet E.U.A. 3 545 583). Une méthode dé synthèse à partir de l'énamme (I) ci-dessous et d'$\alpha$-bromométhyl-acrylate de méthyle est décrite dans le brevet français n° 2 190 112.

La vincamine est connue pour ses propriétés pharmacologiques intéressantes en tant qu'oxygénateur cérébral et vasorégulateur cérébral et pour son activité thérapeutique pour le traitement des insuffisances cérébrales.

La synthèse de l'invention est effectuée à partir de l'éthyl-1 hexahydro-2,3,4,6,7,12 indolo[2,3a]quinolizine,

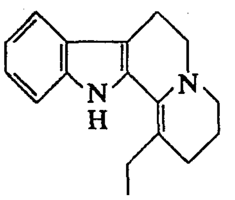

(1)

produit décrit par WENKERT et WICKBERG (J. Am. Chem. Soc. 87, 1580 (1965)).

Le schéma (1) de synthèse de la vincamine et le schéma (2) de synthèse de la désoxyvincamine et de l'apovincamine sont représentés aux pages suivantes.

Schéma 1

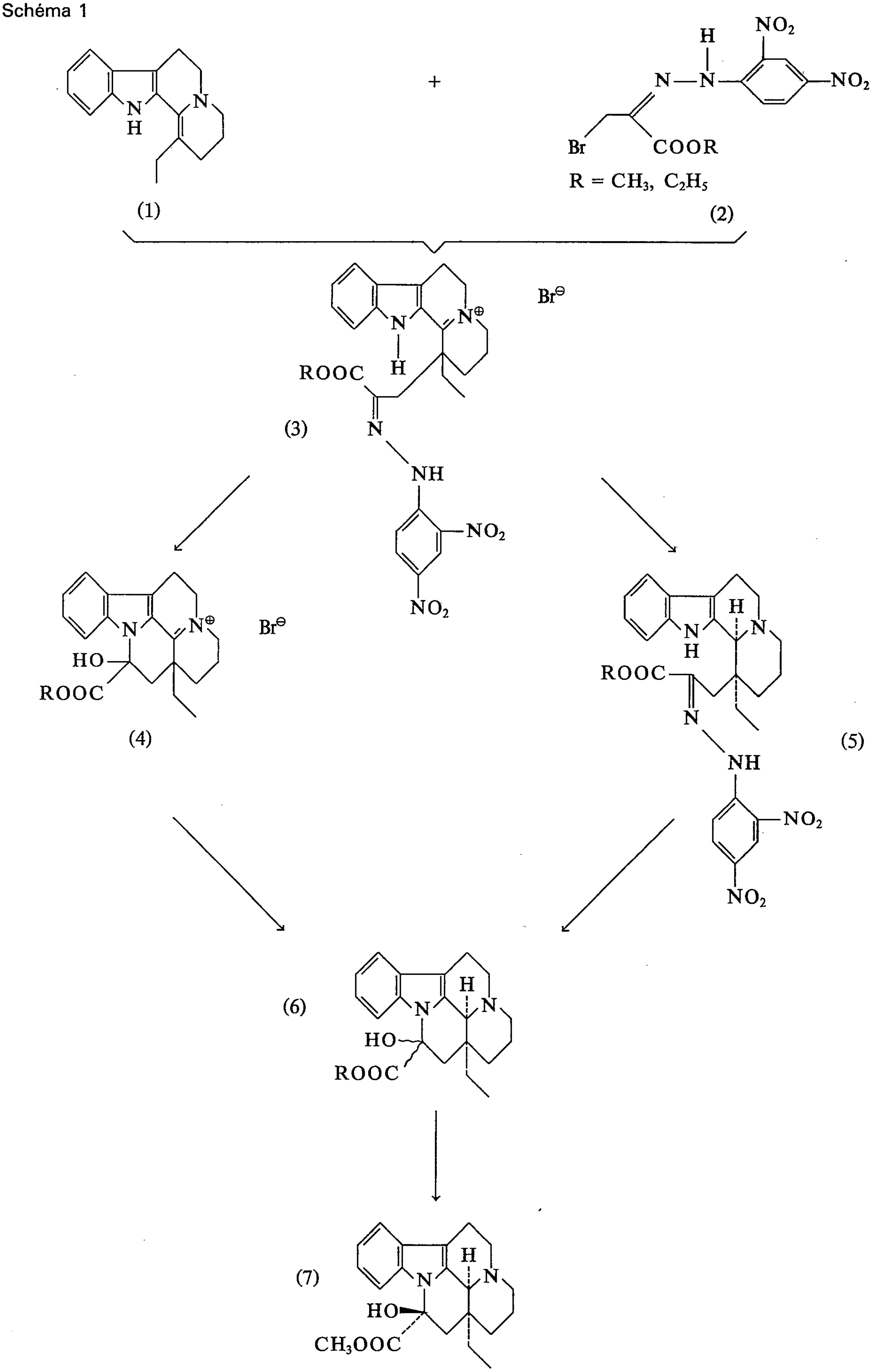

(1) + (2)

R = $CH_3$, $C_2H_5$

(3)

(4)

(5)

(6)

(7)

Schéma 2

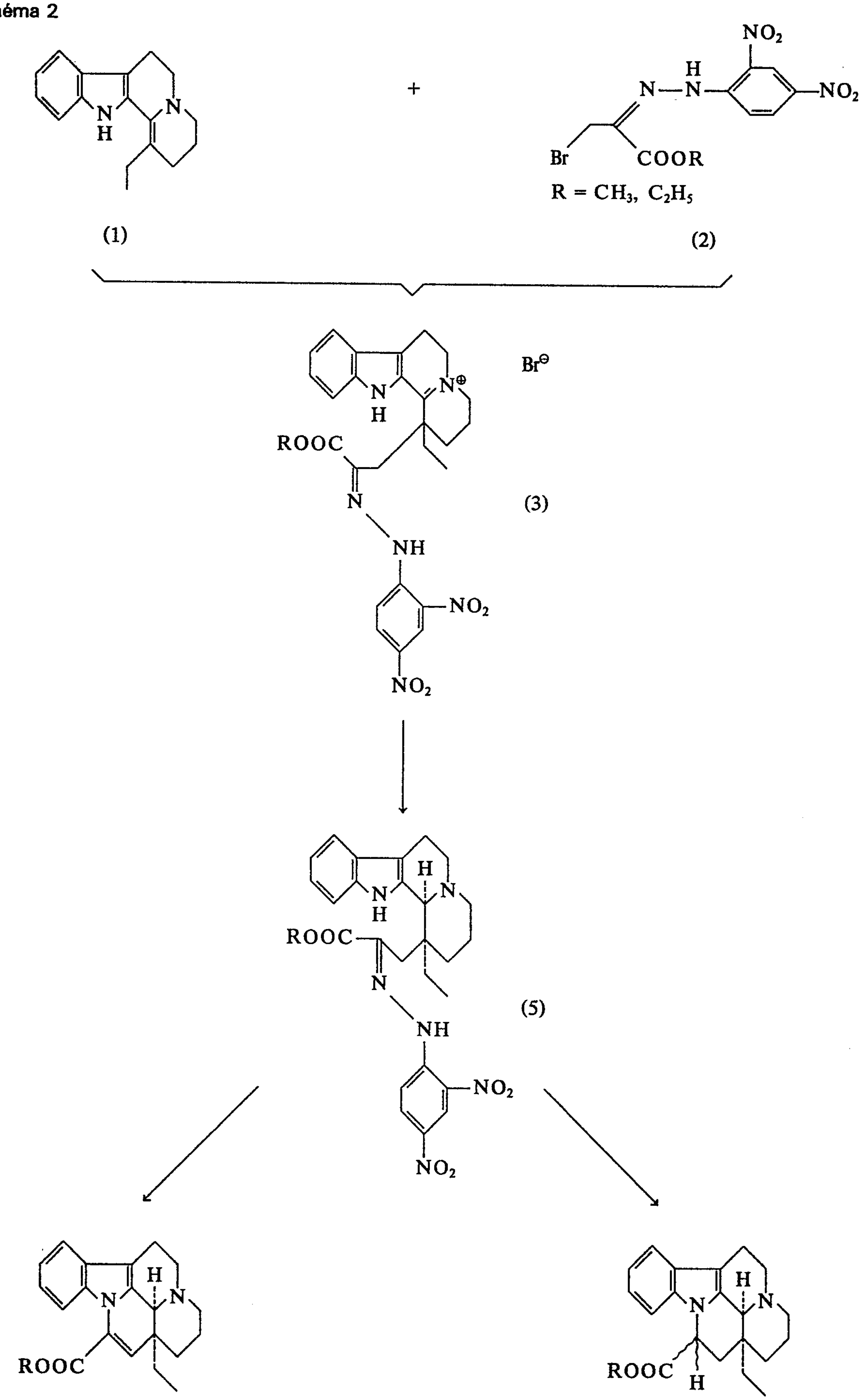

R = $CH_3$, $C_2H_5$

(1)

(2)

(3)

(5)

Pour préparer la vincamine, selon l'invention, on fait réagir l'énamine (1) avec la (dinitro-2,4 phényl)-hydrazone du bromopyruvate de méthyle ou d'éthyle (2), puis soit on déprotège le composé (3) pour obtenir le composé cyclisé (4) que l'on réduit pour obtenir le composé (6), soit on réduit le composé (3) pour obtenir le composé (5) que l'on déprotège pour obtenir le composé cyclisé (6) et éventuellement on transestérifie le composé (6) (s'il s'agit de l'ester éthylique) en (±)-vincamine (7).

La première étape est effectuée dans un solvant tel que l'acétate d'éthyle à la température ambiante. La déprotection du composé (3) en composé (4) est effectuée dans un solvant tel qu'un mélange d'acétonitrile et d'eau ajusté à pH 8 à l'aide de borate de sodium et d'acide chlorhydrique, à la température ambiante.

La réduction du composé (4) peut être réalisée par tout moyen approprié, tel que le zinc en présence d'acide acétique à 50% ou le nickel de Raney dans de l'acide acétique à 50%.

La réduction du composé (3) en composé (5) peut être réalisée par exemple, par un hydrure alcalin en milieu acide dans un solvant tel que méthanol ou acétonitrile.

La déprotection du composé (5) est réalisée par le chlorure de titane III dans un solvant tel que le méthanol ou l'acétonitrile contenant du formaldéhyde et un acide tel que l'acide chlorhydrique ou l'acide acétique dans un domaine de températures allant de 20 à 140° C.

La transestérification éventuelle du composé (6) en (±) vincamine (7) est effectuée par chauffage dans du méthanol à la température de reflux en présence de méthylate de sodium.

La cis-vincamine obtenue selon la synthèse de l'invention est sous forme racémique (±). Le composé (5) est obtenu directement sous la forme cis à partir du composé (3), dans une proportion d'au moins 80%.

Le composé (6) est un mélange des deux épimères sur le carbone en position 14, le proton en 3 et l'éthyle en 6 étant en position relative cis.

Pour préparer l'apovincamine et la désoxyvincamine, selon l'invention, on fait réagir l'énamine (1) avec la (dinitro-2,4 phényl)-hydrazone du bromopyruvate d'éthyle ou de méthyle (2) puis on réduit le composé (3) en composé (5) que l'on fait réagir dans de l'acide formique soit avec une solution de chlorure de titane III à 15% afin d'obtenir l'apovincaminate d'éthyle ($R=C_2H_5$) ou l'apovincamine ($R=CH_3$) soit une solution de chlorure de titane III à 30% pour obtenir le désoxyvincaminate d'éthyle ($R=C_2H_5$) ou la désoxyvincamine ($R=CH_3$).

La vincamine obtenue selon le schéma réactionnel 1 est la vincamine cis sous forme racémique (±). Or, la Demanderesse a réussi à dédoubler le composé (5) (lui aussi sous forme racémique) pour obtenir les énantiomères dextrogyre et lévogyre et aboutir directement à partir de ce composé à la cis (+)vincamine et à la cis (−)vincamine.

Selon l'invention, on réalise le dédoublement du composé (5) à l'aide d'un acide optiquement actif, tel que l'acide dibenzoyl-L-tartrique dans un solvant, tel que l'acétonitrile.

Les exemples suivants illustrent l'invention.

Les analyses et les spectres IR et RMN ont confirmé la structure des composés.

Le composé de départ (2) est nouveau. Il est obtenu de la manière suivante lorsque R est $C_2H_5$:

On ajoute 39 g (0,2 mole) de bromopyruvate d'éthyle à 41 g (0,2 mole) de (dinitro-2,4 phényl)-hydrazine dans un acide tel que l'acide chlorhydrique ou acétique. On laisse revenir la température à 20° C, filtre le précipité formé, lave abondamment à l'eau puis sèche sous vide à 60° C.

On obtient 60 g de [(dinitro-2,4 phényl)-hydrazono-2 bromo-3 pyruvate] d'éthyle.

F = 150,5° C.

On prépare de la même manière le [(dinitro-2,4 phényl)-hydrazono-2 bromo-3 pyruvate] de méthyle.

F = 158° C.

## Exemple 1

### (±)-vincamine · Schéma 1 · $R=C_2H_5$

1. Bromure de [(dinitro-2,4 phényl)-hydrazono-2 éthoxycarbonyl-2 éthyl]-1 éthyl-1 hexahydro-1,2,3,4,6,7 (12H) indolo[2,3a]quinolizine-5-ium (composé 3)

On dissout 29 g (77,35 mmole) du composé de départ (2) dans 1,7 l d'acétate d'éthyle. On y ajoute, tout en agitant, 8,95 g (88,6 mmole) de triéthylamine, séchée sur KOH, puis on ajoute une solution de 15,7 g (62,4 mmole) de l'énamine (1) dans 500 ml d'acétate d'éthyle. On agite le mélange réactionnel à la température ambiante pendant la nuit. On filtre le précipité formé et on le lave avec de l'acétate d'éthyle. On sèche le produit obtenu sous vide à 40° C. On obtient 39 g (rendement 99,6%) d'une poudre orange qui fond à 200° C avec décomposition.

2. Chlorure de déhydro-vincaminate d'éthyle (composé 4)

On dissout 1,56 g (2,48 mmole) du composé (3) précédemment obtenu dans 50 ml d'acétonitrile. On joute 150 ml d'eau puis 50 ml d'un tampon à pH 8 constitué par une solution concentrée de borate de sodium et d'acide chlorhydrique. On agite le mélange réactionnel pendant une nuit. On filtre le léger précipité formé. On lave la phase aqueuse, trois fois, avec 50 ml de toluène et on l'extrait avec du chlorure de méthylène. On sèche la phase organique sur du sulfate de sodium, on filtre et concentre. On obtient 1 g (rendement 100%) du composé (4) que l'on utilise tel quel pour l'étape suivante.

3. (±)-vincamine

On dissout 1 g (2,48 mmole) du composé (4) précédemment obtenu dans 65 ml d'acide acétique à 50%. On porte la solution à 88° C pendant 4 mn, puis on ajoute 5 g de zinc, par petites quantités. On maintient le chauffage pendant 5 mn. On verse le mélange réactionnel sur 65 g de glace et le traite par 50 ml d'ammoniaque à 28%. On filtre le précipité et on le sèche sous vide à 60° C. On obtient 0,55 g de composé (6) que l'on reprend par 3 ml de méthanol. On chauffe le mélange à la température de reflux en présence de méthylate de sodium (0,1 ml d'une solution à 30% dans le méthanol) pendant 5 h. On obitent 0,33 g (rendement 38%) de (±)-vincamine.

Exemple 2

(±)-vincamine (Schéma 1 · R = $C_2H_5$)

1. Le composé (3) est préparé comme dans l'exemple 1
2. [(dinitro-2,4 phényl)-hydrazono-2 éthoxycarbonyl-2 éthyl]-1 éthyl-1 octahydro-1,2,3,4,6,7,12,12b indolo[2,3a]-quinolizine

On ajoute 10,5 g (167 mmole) de $NaBH_3CN$, tout en agitant, à une solution de 35 g de composé (3) dans 40 ml d'acide acétique, 200 ml de méthanol, 200 ml d'acétonitrile et 200 ml d'eau. Après une heure à la température ambiante, on traite la solution avec 80 ml d'ammoniaque à 28% et 200 ml d'eau. On extrait la phase aqueuse avec du chlorure de méthylène. Les phases organiques sont concentrées puis reprises par 150 ml de méthanol. On porte le mélange à la température de reflux pendant 15 mn, laisse refroidir et filtre.
On obtient 24,57 g (rendement 80%) de composé (5) fondant à 214° C.

3. (±) Vincaminate d'éthyle

On dissout 1 g (1,82 mmole) du composé (5) obtenu précédemment dans 20 ml d'acétone et 10 ml d'acide acétique. On dégaze la solution à l'aide d'argon et on l'ajoute en 2 mn, à une solution maintenue à 67° C, contenant 30 ml de chlorure de titane à 15% dans de l'eau, 30 ml de formaldéhyde à 37% dans de l'eau dégazée et 10 ml d'acide acétique dégazé.
Après 20 mn de réaction à 67° C, on ajoute 200 ml d'eau glacée au mélange réactionnel que l'on extrait avec du chlorure de méthylène. Les phases organiques réunies sont concentrées. On reprend le résidu par 30 ml d'eau glacée. On traite la solution obtenue avec de l'ammoniaque à 28%. On filtre et sèche le précipité à 60° C, sous vide. On obtient 0,460 g (68%) du mélange d'épimères (en position 14) du composé (6).

4. (±)-vincamine

On chauffe le composé (6) en solution dans du méthanol en présence de méthylate de sodium à la température de reflux pendant 5 h. On obtient 0,326 g de (±)-vincamine.

Analyse spectrographique

RMN: ($CDCl_3$, DMSO), 0,95 (t, 3H), 1,40 (M, 6H), 2,60 (m, 3H), 3,30 (m, 2H), 3,78 (s, 3H), 3,86 (large S, 1H), 7,07 (m, 3H), 7,36 (m, 1H)
IR: KBr, $cm^{-1}$) 3440 (OH, large), 2920 (m), 2840 (sh), 1730 (COOMe)

Exemple 3

(±)-vincamine (Schéma 1 · R=$CH_3$)

1. On ajoute une solution contenant 6,88 g d'énamine (27 mM) et 2,9 g de triéthylamine (29 mM) dans 60 ml d'acétate d'éthyle à une suspension de 10 g de (dinitro-2,4 phényl)-hydrazone du bromopyruvate de méthyle (29 mM) dans 140 ml d'acétate d'éthyle. On agite pendant 16 h, filtre et lave avec de l'acétate d'éthyle. On sèche sous vide à 70°C. On obtient 15,2 g de bromure de [(dinitro-2,4 phényl)-hydrazono-2 méthoxy-carbonyl-2 éthyl]-1 éthyl-1 hexahydro-1,2,3,4,6,7 (12H) indolo[2,3a]-quinolizine-5-ium (Rdt: 92%).

   F=205°C (déc.)

2. On réduit 4,9 g (7,98 mM) de ce dernier composé avec 1,29 g (24 mM) de borohydrure de potassium pour obtenir la [(dinitro-2,4 phényl)-hydrazono-2 méthoxycarbonyl-2 éthyl]-1 éthyl-1 octahydro-1,2,3,4,6,7,12b indolo[2,3a]-quinolizine (3,6 g; Rdt: 74%).

   F=205–206°C.

3. 2 g (2,74 mM) de produit précédemment obtenu dans 28 ml d'acétone sont ajoutés à une solution à 55–60°C ontenant 60 ml d'acétone, 10 ml d'une solution aqueuse de formol à 37%, 20 ml d'acide acétique et 60 ml de chlorure de titane III (15%). La réaction est maintenue à 60°C pendant 15 minutes, puis additionnée de glace. On ajoute 5 g de $NaNO_2$ (72 mM) et fait barboter de l'azote pendant 15 minutes. On extrait au chlorure de méthylène, concentre la phase organique et reprend le résidu par 10 ml d'eau. On basifie la solution à l'ammoniaque, filtre et sèche la (±)-vincamine (0,974 g; Rdt: 74%).

   F=242°C (MeOH)

Exemple 4

(±) Apovincaminate d'éthyle

1. Bromure de [(dinitro-2,4 phényl)-hydrazono-2 éthoxycarbonyl-2 éthyl]-1 éthyl-1 hexahydro-1,2,3,4,6,7 (12H) indolo-[2,3a]-quinolizine-5-ium (composé 3)

   On dissout 29 g (77,35 mmole) du composé de départ (2) dans 1,7 l d'acétate d'éthyle. On y ajoute, tout en agitant, 8,95 g (88,6 mmole) de triéthylamine, séchée sur KOH, puis on ajoute une solution de 15,7 g (62,4 mmole) de l'énamine (1) dans 500 ml d'acétate d'éthyle. On agite le mélange réactionnel à la température ambiante pendant la nuit. On filtre le précipité formé et on le lave avec de l'acétate d'éthyle. On sèche le produit obtenu sous vide à 40°C. On obtient 39 g (rendement 99,6%) d'une poudre orange qui fond à 200°C avec décomposition.

2. (±) [(dinitro-2,4 phényl)-hydrazono-2 éthoxycarbonyl-2 éthyl]-1 éthyl-1 octahydro-1,2,3,4,6,7,12,12b indolo[2,3a]-quinolizine

   On ajoute 10,5 g (167 mmole) de $NaBH_3CN$, tout en agitant, à une solution de 35 g de composé (3) dans 40 ml d'acide acétique, 200 ml de méthanol, 200 ml d'acétonitrile et 200 ml d'eau. Après une heure à la température ambiante, on traite la solution avec 80 ml d'ammoniaque à 28% et 200 ml d'eau. On extrait la phase aqueuse avec du chlorure de méthylène. Les phases organiques sont concentrées puis reprises par 150 ml de méthanol. On porte le mélange à la température de reflux pendant 15 mn, laisse refroidir et filtre.
   On obtient 24,57 g (rendement 80%) de composé (5) fondant à 214°C.

3. (±) Apovincaminate d'éthyle

   On porte à la température de reflux 2,5 g (4,5 mmole) du composé obtenu précédemment dans 75 ml d'acide formique et on ajoute 60 ml d'une solution de chlorure de titane à 15%. On chauffe le mélange réactionnel pendant 20 mn à reflux puis on ajoute de la glace. On élimine le dioxyde de titane par filtration et on extrait le produit à l'aide de dichlorométhane. Les phases organiques sont lavées à l'ammoniaque puis à l'eau puis séchées sur sulfate de sodium. On élimine le solvant,

et recristallise le résidu solide dans l'éther de pétrole. On obtient 1,24 g d'apovincaminate d'éthyle sous la forme d'un solide jaune pâle.

F = 122° C.

On peut de la même manière préparer l' (±) apovincamine à partir du composé (3) obtenu à partir du bromopyruvate de méthyle et de l'énamine (1).

Exemple 5

(±) Désoxyvincaminate d'éthyle

On porte à la températur du reflux 5 g (9 mmole) de composé obtenu dans l'exemple 4, paragraphe 1, en solution dans 150 ml d'acide formique et on ajoute 125 ml d'une solution à 30% de chlorure de titane III. Le reflux est maintenu pendant 20 mn. On ajoute de la glace au mélange réactionnel et on élimine le précipité de dioxyde de titane par filtration. On extrait le produit du filtrat à l'aide de dichlorométhane et lave la phase organique à l'ammoniaque et à l'eau. Après séchage sur sulfate de sodium et élimination du solvant, on obtient une huile visqueuse (3,2 g), constituée uniquement du mélange des deux épimères en $C_{14}$ du désoxyvincaminate d'éthyle (données spectrales et chromatographiques conformes) (Rdt: 100%).

On peut de la même manière, préparer la (±) désoxyvincamine à partir du bromopyruvate de méthyle et de l'énamine (1).

Exemple 6

(+) Vincamine

1. ⊕ [(dinitro-2,4 phényl)-hydrazono-2 méthoxycarbonyl-2 éthyl]-1β éthyl-1α octahydro-1,2,3,4,6,7,12,12bα indolo[2,3a]-quinolizine
et
⊖ [(dinitro-2,4 phényl)-hydrazono-2 méthoxycarbonyl-2 éthyl]-1α éthyl-1β octahydro-1,2,3,4,6,7,12,12bβ indolo[2,3a]-quinolizine

On porte à la température du reflux 100 ml (0,0187 mM) de composé (5) et 70 mg d'acide dibenzoyl-L-tartrique dans 3 ml de $CH_3CN$. Après refroidissement, on filtre les cristaux formés et lave à l'acétonitrile (1 ml). Le filtrat est concentré, traité à l'ammoniaque diluée et extrait au chlorure de méthylène. On lave les phases organiques à l'eau, sèche sur $Na_2SO_4$ et concentre. Le résidu est repris par 2 ml d'acétonitrile. On laisse cristalliser la solution pendant 24 h et filtre les cristaux formés. Ces cristaux sont constitués par 46 mg de l'énantiomère lévogyre du composé (5) dont le pouvoir rotatoire $[\alpha]_D^{20} = +77,9°$ (c = 0,1; AcOH).

F = 195 – 197° C.

Le filtrat est concentré et le résidu est cristallisé dans 3 ml de MeOH. On obtient 36 mg de l'énantiomère dextrogyre du composé (5) dont le pouvoir rotatoire $[\alpha]_D^{20} = +77,9°$ (c = 0,1; AcOH).

F = 195 – 197° C.

2. (+) Vincamine

Dans un ballon on introduit 6 ml de formol à 37% et 5 ml de chlorure de titane III (15%). On porte à 60° C. On ajoute une solution de 310 mg de l'énantiomère (+) du composé 5 dans 2 ml d'acétone et 2 ml d'acide acétique. On laisse réagir pendant 30 mn à 55 – 60° C. On traite par $NaNO_2$. On fait passer un courant d'argon pendant 3 mn. On extrait au chlorure de méthylène plusieurs fois. On concentre et on reprend par de la glace et une solution ammoniacale. On filtre et recristallise dans le méthanol en présence de méthylate de sodium. On obtient la (+) vincamine qui fond à 227 – 230° C.

$[\alpha]_D^{20} = +41°$ (c = 1; pyridine)

3. (−) Vincamine

On fait réagir l'énantiomère lévogyre du composé (5) dans les mêmes conditions que dans le paragraphe 2 et on obtient la (−) vincamine.

F = 228 − 230° C.
$[\alpha]_D^{20}$ = −41,4° (c = 1; pyridine)

**Revendications**

1. Procédé de préparatior de dérivés vincaminiques, en particulier de la vincamine, de l'apovincamine et de la désoxyvincamine, procédé caractérisé en ce que l'on fait réagir l'énamine (1)

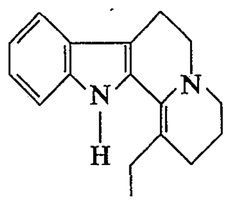

(1)

avec la (dinitro-2,4phényl)-hydrazone du bromopyruvate d'éthyle ou de méthyle (2)

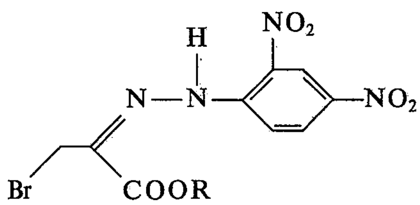

(2)

(R = $CH_3$ ou $C_2H_5$)

puis soit on déprotège le composé (3)

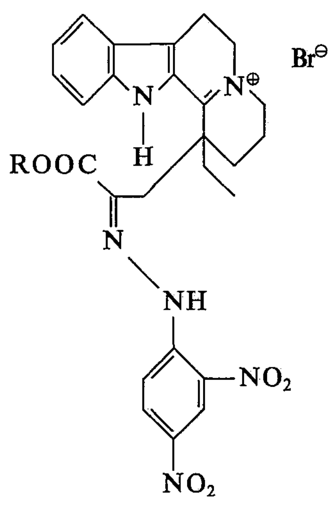

(3)

pour obtenir le composé cyclisé (4)

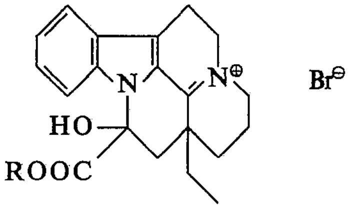

(4)

que l'on réduit pour obtenir le composé (6)

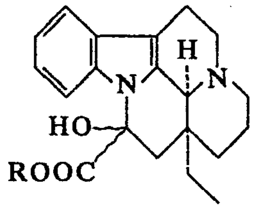

(6)

soit on réduit le composé (3)

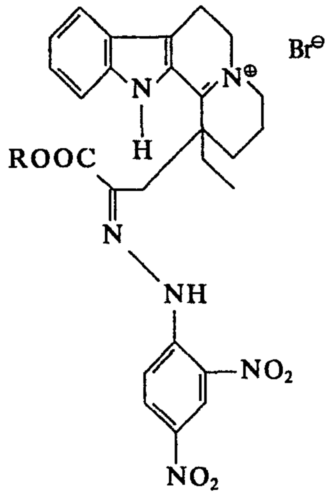

(3)

pour obtenir le composé (5)

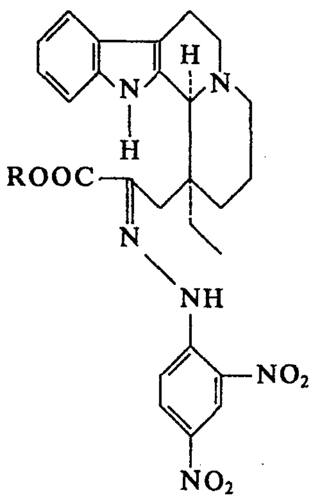

(5)

composé (5) que

– soit l'on déprotège pour obtenir le composé cyclisé (6)

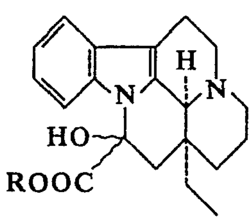

(6)

que l'on transestérifie éventuellement lorque R est $C_2H_5$ en (±)-vincamine (7)

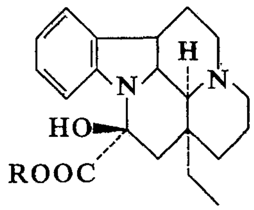

(7)

– soit l'on fait réagir dans de l'acide formique ou bien avec une solution de chlorure de titane III à 15% afin d'obtenir l'apovincaminate d'éthyle ($R = C_5H_5$) ou l'apovincamine ($R = CH_3$) ou bien avec une solution de chlorure de titane III à 30% pour obtenir le désoxyvincaminate d'éthyle ($R = C_2H_5$) ou la désoxyvincamine ($R = CH_3$).

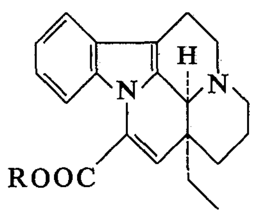

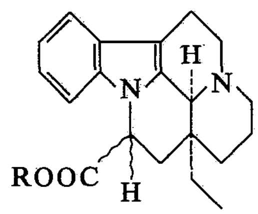

2. Procédé selon la revendication 1, procédé caractérisé en ce que l'on prépare le composé (3) dans de l'acétate d'éthyle à la température ambiante, on déprotège le composé (3) à l'aide de borate de sodium et d'acide chlorhydrique dans un mélange acétonitrile/eau à la température ambiante, on réduit le composé (4) à l'aide de zinc ou de nickel de Raney dans de l'acide acétique, et on transestérifie éventuellement le composé (6) en (±)-vincamine par chauffage dans du méthanol à la température de reflux en présence d'un méthylate alcalin.

3. Procédé selon la revendication 1, caractérisé en ce que l'on prépare le composé (3) dans de l'acétate d'éthyle à la température ambiante, on réduit le composé (3) en composé (5) à l'aide d'un hydrure alcalin en milieu acide dans un mélange de solvants méthanol/acétonitrile, on déprotège le composé (5) à l'aide de chlorure de titane III dans un solvant tel que le méthanol ou l'acétonitrile contenant du formaldéhyde et de l'acide chlorhydrique ou acétique à une température allant de 20 à 140°C, et on transestérifie éventuellement le composé (6) en (±)-vincamine par chauffage dans du méthanol à la température de reflux en présence d'un méthylate alcalin.

4. Procédé selon la revendication 1, caractérisé en ce que l'on prépare le composé (3) dans de l'acétate d'éthyle à la température ambiante, on réduit le composé (3) en composé (5) à l'aide d'un hydrure alcalin en milieu acide dans des solvants tels que méthanol ou acétonitrile, puis on fait réagir le composé (5) avec une solution de chlorure de titane III à 15% afin d'obtenir l' (±) apovincaminate d'éthyle ($R = C_2H_5$) ou l' (±) apovincamine ($R = CH_3$).

5. Procédé selon la revendication 1, caractérisé en ce que l'on prépare le composé (3) dans de l'acétate d'éthyle à la température ambiante, on réduit le composé (3) en composé (5) à l'aide d'un hydrure alcalin en milieu acide dans des solvants tels que méthanol ou acétonitrile, puis on fait réagir le composé (5) avec une solution de chlorure de titane III à 30% afin d'obtenir le (±) désoxyvincaminate d'éthyle ($R = C_2H_5$) ou la (±) désoxyvincamine ($R = CH_3$).

6. Procédé de préparation de la (+) ou (−) vincamine selon la revendication 1, procédé caractérisé en ce que l'on fait réagir le composé (5) dans lequel $R = CH_3$ à l'aide d'un acide optiquement actif afin d'obtenir les énantiomères dextrogyre et levogyre que l'on fait réagir avec du chlorure de titane III pour obtenir la (+)-vincamine et la (−)-vincamine.

7. Procédé selon la revendication 6, caractérisé par le fait que l'acide optiquement actif est l'acide dibenzoyl-L-tartrique.

**Patentansprüche**

1. Verfahren zur Herstellung von Vincaminverbindungen, insbesondere von Vincamin, Apovincamin und Desoxyvincamin, dadurch gekennzeichnet, daß man das Enamin (1)

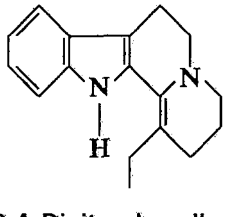

(I)

mit dem 2,4-Dinitrophenylhydrazon des Äthyl- oder Methylbrompyruvats (2)

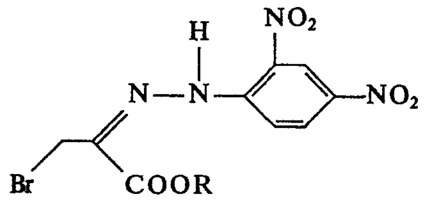

(2)

(R = $CH_3$ oder $C_2H_5$)

umsetzt, dann entweder die Verbindung (3)

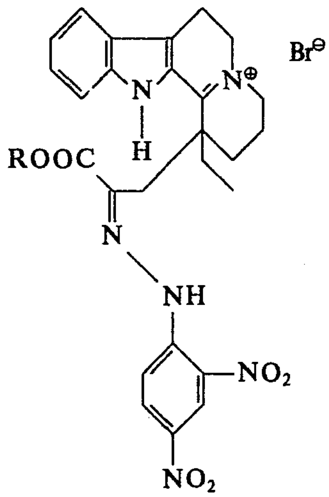

(3)

von ihrer Schutzgruppe befreit, um die zyklisierte Verbindung (4)

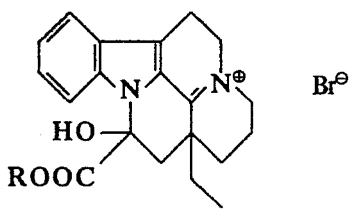

(4)

zu gewinnen, die man zur Herstellung der Verbindung (6)

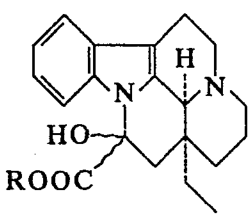

(6)

reduziert, oder die Verbindung (3)

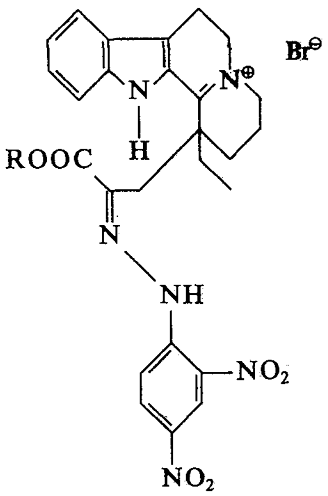

(3)

reduziert, um die Verbindung (5)

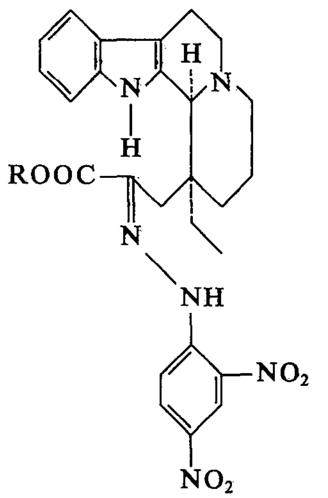

(5)

zu erhalten, die man

– entweder von der Schutzgruppe befreit, um die zyklisierte Verbindung (6)

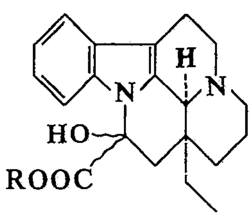

(6)

zu gewinnen, die man gegebenenfalls, wenn R für $C_2H_5$ steht, zum (±)-Vincamin (7)

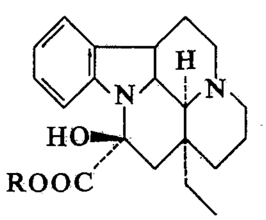

(7)

umestert,

– oder die man in Ameisensäure entweder mit einer 15%igen Titan(III)-chloridlösung zur Gewinnung von Äthyl-Apovincaminat ($R = C_2H_5$) oder von Apovincamin ($R = CH_3$) oder mit einer 30%igen Titan(III)-chloridlösung zur Gewinnung von Äthyl-Desoxyvincaminat ($R = C_2H_5$) oder von Desoxyvincamin ($R = CH_3$) umsetzt.

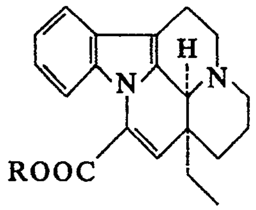

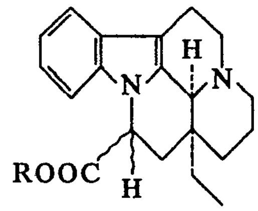

2. Verfahren nach Anspruch 1, dadurch gekennzeichnet, daß man die Verbindung (3) bei Raumtemperatur in Äthylacetat herstellt, die Verbindung (3) mit Hilfe von Natriumborat und Chlorwasserstoffsäure in einer Mischung von Acetonitril/Wasser bei Raumtemperatur von der Schutzgruppe befreit, die Verbindung (4) mit Hilfe von Zink oder Raney-Nickel in Essigsäure reduziert und gegebenenfalls die Verbindung (6) durch Erhitzen in Methanol auf Rückflußtemperatur in Gegenwart eines Alkalimethylats umestert.

3. Verfahren nach Anspruch 1, dadurch gekennzeichnet, daß man die Verbindung (3) bei Raumtemperatur in Äthylacetat herstellt, die Verbindung (3) zur Verbindung (5) mit Hilfe eines Alkalihydrids in saurem Milieu in einer Mischung der Lösungsmittel Methanol/Acetonitril reduziert, an der Verbindung (5) mit Hilfe von Titan(III)-chlorid in einem Lösungsmittel wie Methanol oder Acetonitril mit einem Gehalt von Formaldehyd und Chlorwasserstoffsäure oder Essigsäure bei einer Temperatur von 20 bis 140° die Schutzgruppe abspaltet, und gegebenenfalls die Verbindung (6) durch Erhitzen in Methanol bei Rückflußtemperatur in Gegenwart eines Alkalimethylats zum (±)-Vincamin umestert.

4. Verfahren nach Anspruch 1, dadurch gekennzeichnet, daß man die Verbindung (3) bei Raumtemperatur in Äthylacetat herstellt, die Verbindung (3) mit Hilfe eines Alkalihydrids in saurem Milieu in Lösungsmitteln wie Methanol oder Acetonitril zur Verbindung (5) reduziert und dann die Verbindung (5) mit einer 15%igen Lösung von Titan(III)-chlorid reagieren läßt, um das Äthyl-(±)-Apovincaminat ($R = C_2H_5$) oder das (±)-Apovincamin ($R = CH_3$) zu erhalten.

5. Verfahren nach Anspruch 1, dadurch gekennzeichnet, daß man die Verbindung (3) bei Raumtemperatur in Äthylacetat herstellt, die Verbindung (3) mit Hilfe eines Alkalihydrids in saurem Milieu in Lösungsmitteln wie Methanol oder Acetonitril zur Verbindung (5) reduziert und dann die Verbindung (5) mit einer 30%igen Titan(III)-chloridlösung reagieren läßt, um das Äthyl-(±)Desoxyvincaminat ($R = C_2H_5$) oder das (±)Desoxyvincamin ($R = CH_3$) zu erhalten.

6. Verfahren zur Herstellung des (+)- oder (−)-Vincamins nach Anspruch 1, dadurch gekennzeichnet, daß man die Verbindung (5), in welcher $R = CH_3$, mit Hilfe einer optisch aktiven Säure umsetzt, um die rechtsdrehenden oder linksdrehenden Enantiomeren zu erhalten, die man zur Gewinnung des (+)-Vincamins oder des (−)-Vincamins mit Titan(III)-chlorid reagieren läßt.

7. Verfahren nach Anspruch 6, dadurch gekennzeichnet, daß die optisch aktive Säure die Dibenzoyl-L-weinsäure ist.

**Claims**

1. A process for the preparation of vincamine derivatives, in particular of vincamine, apovincamine and deoxyvincamine, characterized in that an enamine (1).

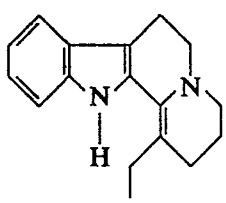

(1)

is reacted with the (2,4-dinitro-phenyl)hydrazone of ethyl- or methyl bromopyruvate (2)

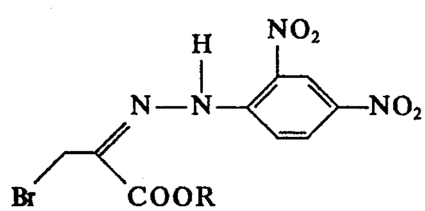

(2)

($R = CH_3$ or $C_2H_5$)

and then the compound (3)

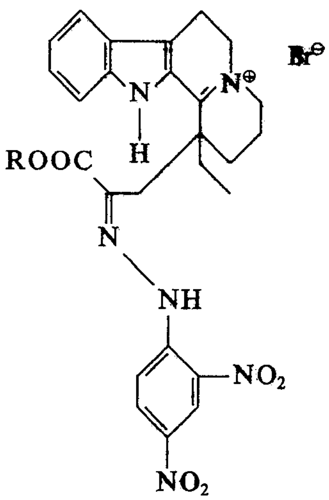

(3)

is either deprotected to produce the cyclized compound (4)

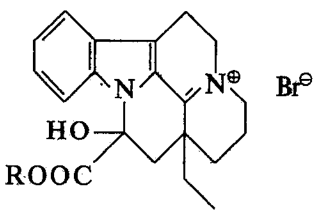

(4)

which is reduced to produce compound (6)

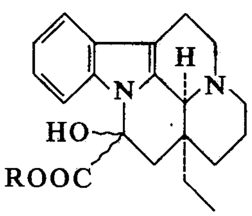

(6)

or the compound (3)

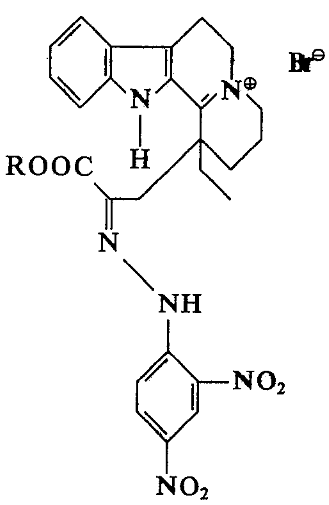

(3)

is reduced to produce compound (5)

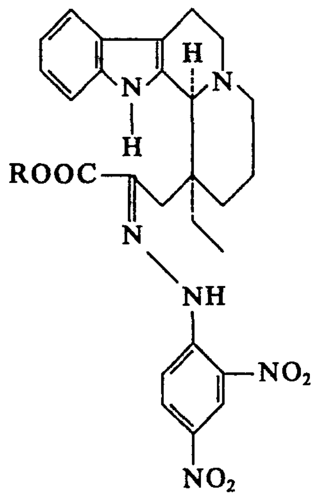

(5)

and said compound (5) is

– either deprotected to produce the cyclized compound (6)

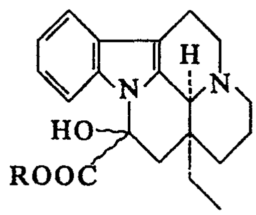

(6)

which is optionally transesterified if R is $C_2H_5$ to produce (±) vincamine (7)

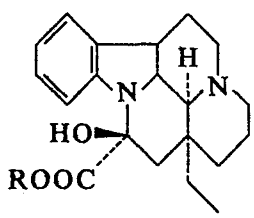

(7)

– or reacted in formic acid or in a 15% solution of titanium III chloride to produce ethyl apovincaminate (R = $C_2H_5$) or apovincamine (R = $CH_3$), or in a 30% solution of titanium III chloride to produce ethyl desoxyvincaminate (R = $C_2H_5$) or deoxyvincamine (R = $CH_3$)

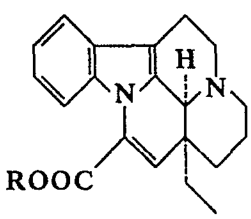

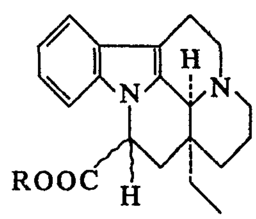

2. A process according to claim 1, characterized in that compound (3) is prepared in ethyl acetate at ambient temperature, compund (3) is deprotected by means of sodium borate and hydrochloric acid in a mixture of acetonitrile/water at ambient temperature, compound (4) is reduced by means of zinc or Raney nickel in acetic acid, and compound (6) is optionally transesterified into (±) vincamine by heating in methanol at reflux temperature in the presence of an alcali metal methylate.

3. A process according to claim 1, characterized in that compound (3) is prepared in ethyl acetate at ambient temperature, compound (3) is reduced to compound (5) by means of an alcali metal hydride in acid medium in a solvent mixture of methanol/acetonitrile, compound (5) is deprotected by means of titanium III chloride in a solvent such as methanol or acetonitrile containing formaldehyde and hydrochlorid or acetic acid at a temperatur of from 20 to 140°C, and compound (6) is optionally transesterified into (±) vincamine by heating in methanol at reflux temperature in the presence of an alcali metal methylate.

4. A process according to claim 1, chracterized in that compound (3) is prepared in ethyl acetate at

ambient temperature, compound (3) is reduced to compound (5) by means of an alcali metal hydride in acid medium in solvents such as methanol or acetonitrile, and then compound (5) is reacted with a 15% solution of titanium III chloride so as to produce ethyl (±) apovincaminate ($R=C_2H_5$) or (±) apovincamine ($R=CH_3$).

5. A process according to claim 1, characterized in that compound (3) is prepared in ethyl acetate at ambient temperature, compound (3) is reduced to compound (5) by means of an alcali metal hydride in acid medium in solvents such as methanol or acetonitrile, and then compound (5) is reacted with a 30% solution of titanium III chloride so as to produce ethyl (+) deoxyvincaminate ($R=C_2H_5$) or (±) deoxyvincamine ($R=CH_3$).

6. A process for the preparation of (+) or (−) vincamine according to claim 1, characterized in that compound (5), wherein $R=CH_3$, is reacted with an optically active acid so as to produce the destrogyre or levogyre enantiomers which are reacted with titanium III chloride to produce (+) vincamine and (−) vincamine.

7. A process according to claim 6, characterized in that the optically active acid is dibenzoyl-L-tartaric acid.